# EUROPÄISCHE PATENTSCHRIFT

(11) **EP 0 906 259 B1**
(45) Veröffentlichungstag und Bekanntmachung des Hinweises auf die Patenterteilung: **28.03.2001**
(21) Anmeldenummer: 97927114.5
(22) Anmeldetag: 03.06.1997
(51) Int. Cl.: C07C 29/20, C07C 35/21

(54) **VERFAHREN ZUR HERSTELLUNG VON ISOCAMPHYL-CYCLOHEXANOLEN**
PROCESS FOR PRODUCING ISOCAMPHYL CYCLOHEXANOLES
PROCEDE DE PRODUCTION D'ISOCAMPHYLCYCLOHEXANOLES

(30) Priorität: 03.06.1996 DE 19622187
(43) Veröffentlichungstag der Anmeldung: 07.04.1999
(73) Patentinhaber: BAYER AG, 51368 Leverkusen (DE)
(72) Erfinder: DARSOW, Gerhard, D-47804 Krefeld (DE); NIEMEIER, Wilfried, D-47803 Krefeld (DE)
(86) Internationale Anmeldenummer: EP9702870
(87) Internationale Veröffentlichungsnummer: WO9746505

(56) Entgegenhaltungen:
- DE-A- 2 921 139
- US-A- 4 014 944
- CHEMICAL ABSTRACTS, vol. 51, no. 21, 10.November 1957 Columbus, Ohio, US; abstract no. 17107c, L.A. KHELFITS, ET AL.: "Industrial method for the manufacture of santalidol" XP002038808 & MASOLBOINO-ZHIROVAYA PROM., Bd. 23, Nr. 6, 1957, Seiten 35-38,

## Beschreibung

Die Erfindung betrifft ein kontinuierliches Verfahren zur Herstellung von Isocamphyl-cyclohexanolen aus Isocamphyl-guajakol oder Isocamphyl-phenol durch Hydrierung mit Wasserstoff bei erhöhter Temperatur und erhöhtem Druck unter Einsatz von trägerfreien reduzierten Formkörpern aus verpreßten Pulvern von Kobalt-, Mangan- und Kupfer(hydr)oxiden und gegebenenfalls von (Hydr)Oxiden der Erdalkalimetalle.

Isocamphyl-cyclohexanole sind Bestandteile der industriell erzeugten Sandelholzduftstoffe, die aus synthetischen Mischungen verschiedener Terpencyclohexanol-Isomeren bestehen. Industrielle Sandelholzduftstoffe ersetzen natürliches Sandelholzöl in Seifen, Kosmetikartikeln und Parfümkompositionen.

Als Ausgangsverbindungen für die Isocamphyl-cyclohexanole können z.B. die Isocamphyl-guajakole in bekannter Weise durch Reaktion von Camphen mit Guajakol unter Mithilfe von sauren Katalysatoren, z.B. Bortrifluorid und Essigsäure, hergestellt werden; die Isocamphyl-guajakole werden dann durch Hydrierung des Aromatkerns und Abspaltung der Methoxygruppe in die Isocamphyl-cyclohexanole verwandelt.

Die Reaktionen verlaufen nach folgender Gleichung: 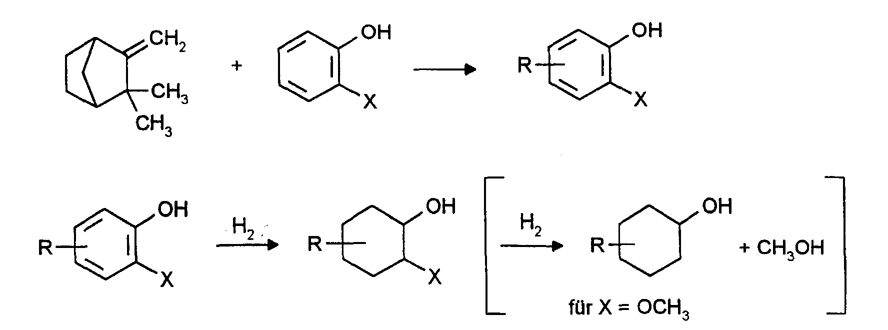 X = OCH₃ oder H

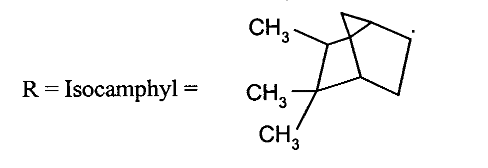

Es ist bereits bekannt, Alkylierungsgemische aus Camphen und Catechol (US 4 014 944) bzw. Camphen und Guajakol (DE-A 2 921 139) mit Wasserstoff über Raney-Nickel zu den gewünschten Isocamphyl-cyclohexanolen zu hydrieren.

Diese Verfahren des Standes der Technik arbeiten ausnahmslos diskontinuierlich mit pulverförmigen Katalysatoren nach dem Suspensionsverfahren.

Bei Pulverkatalysatoren bestehen die folgenden Schwierigkeiten: (1) diese Katalysatoren gezielt und gleichmäßig zu aktivieren, (2) Pulverkatalysatoren mit Hilfe von speziellen Schlammpumpen umzuwälzen und (3) Pulverkatalysatoren vom Reaktionsprodukt quantitativ abzutrennen. Schlammpumpen unterliegen nämlich einer hohen mechanischen Beanspruchung. Die quantitative Entfernung pulverförmiger Katalysatoren ist weiterhin aufwendig, weil sie eine Grob- und eine Feinfiltration mit Apparaten in Wechselausführung erfordert. Ferner ist die Gefahr groß, daß die Katalysatoren durch diese zusätzlichen Operationen schnell ihre Aktivität verlieren und daher weiterhin hohe Katalysatorverbräuche verursachen. Ferner sind Pulverkatalysatoren nur begrenzt belastbar und nur schwierig von den Reaktionsprodukten zu befreien, was ihre Aufarbeitung erschwert.

Es bestand daher der Wunsch ein Verfahren für die Herstellung von Isocamphyl-cyclohexanolen bereitzustellen, wobei hoch belastbare, säurefeste und langlebige Katalysatoren verwendet werden, die frei von komplizierten Trägersystemen und daher wieder aufarbeitbar sein sollten.

Überraschenderweise läßt sich das geschilderte Problem mit Hilfe trägerfreier Festbettkatalysatoren lösen, die durch Reduktion von Formkörpern aus verpreßten Metall(hydr)oxidpulvern erhalten werden können.

Gegenstand der Erfindung ist damit ein kontinuierliches Verfahren zur Herstellung von Isocamphyl-cyclohexanolen aus Verbindungen, die das Kohlenstoffgerüst der Isocamphyl-guajakole bzw. Isocamphyl-phenole besitzen, durch katalytische Hydrierung mit Wasserstoff bei erhöhter Temperatur und erhöhtem Druck, dadurch gekennzeichnet, daß als Katalysatoren trägerfreie Formkörper verwendet werden, die durch Reduktion von Formkörpern aus verpreßten Pulvern von Kobalt-, Mangan-und Kupfer(hydr)oxiden und gegebenenfalls einem oder mehreren (Hydr)Oxiden der Erdalkalimetalle erhältlich sind.

Für die erfindungsgemäß zu verwendenden Katalysatoren betragen (jeweils berechnet als Metall) die Co-Anteile 40 bis 65 Gew.-%, die Mn-Anteile 10 bis 20 Gew.-%, die Cu-Anteile 0,1 bis 0,5 Gew.-% und gegebenenfalls die Erdalkalimetallanteile (bei mehreren in Summe) 0,2 bis 5 Gew.-% des gesamten (Hydr)Oxidpulvers. Der Rest zu 100 Gew.-% ist Sauerstoff für die in oxidischer Form vorliegenden Verbindungen. Ein solcher Katalysator braucht zwar keine (Hydr)Oxide der Erdalkalimetalle enthalten; vorzugsweise besitzt er jedoch einen Gehalt von mindestens einem (Hydr)Oxid der Erdalkalimetalle. Für den Fall des Einsatzes mehrerer (Hydr)Oxide der Erdalkalimetalle liegt jedes dieser (Hydr)Oxide vorzugsweise in einer Menge vor, die nicht kleiner als 20 % und nicht größer als 80 % des genannten Gesamtbereichs von 0,2 bis 5 Gew.-% ist.

Von den Erdalkalielementen eignen sich vor allem Magnesium, Calcium, Strontium und Barium, vorzugsweise Strontium und Barium.

Zur Herstellung der Katalysatoren werden Pulver von Oxiden oder Hydroxiden der genannten Elemente eingesetzt. In bevorzugter Weise werden Oxidpulver der genannten Elemente eingesetzt. Solche Pulver werden mechanisch in solchen Verhältnissen miteinander vermischt, daß sich die oben angegebenen Gewichtsverhältnisse einstellen. Der Rest zu 100 Gew.-% ist stets der Anteil des Sauerstoffs, und alle Gewichtsprozente sind auf das Gesamtgewicht des oxidischen, trägerfreien Formkörpers bezogen. Das Gemisch der Pulver wird sodann auf Tablettier-oder Pelletier-Maschinen unter hohem Druck verpreßt, wobei zur Verbesserung des Haftvermögens der Pulver auch Graphit und/oder Klebstoffe in Mengen von 0,5 bis 1 Gew.-%, bezogen auf das Gesamtgewicht der zu verpressenden Pulver, zum Einsatz kommen können. Beispiele für die Form solcher Preßlinge sind Tabletten, Kugeln oder zylindrische Granulate mit Abmessungen von 1 bis 10 mm, bevorzugt 3 bis 7 mm. Tablettierte Formkörper können weiterhin zur Vergrößerung der äußeren Oberfläche mit einer axialen Durchbohrung versehen sein. Solche verpreßten Formkörper haben makroskopisch betrachtet eine glatte Oberfläche. Die verpreßten Formkörper haben eine hohe Druckfestigkeit auf die Formkörperoberfläche. So haben Tabletten oder zylindrische Granulate auf die ebenen Preßoberflächen eine Druckfestigkeit von 200 bis 800 N/cm², bevorzugt 250 bis 600 N/cm² bei Benutzung eines flächigen Druckstempels, und Tabletten, Kugeln oder zylindrische Granulate eine Druckfestigkeit auf die gewölbten Preßoberflächen von 50 bis 200 N (gemessen als Kraft), bevorzugt 80 bis 140 N bei Benutzung eines messerförmigen Druckgebers. Die innere Oberfläche der eingesetzten verpreßten Formkörper beträgt 30 bis 200 m²/g, bevorzugt 80 bis 160 m²/g. Die Druckfestigkeit der trägerfreien Formkörper kann nach DIN 50 106 bestimmt werden. Die Bestimmung der inneren Oberfläche erfolgt nach F.M. Nelson und F.T. Eggertsen, Analyt. Chem. 30 (1958), S. 1387 bis 1390 bzw. nach S.J. Gregg und K.S.W. Sing, Adsorption Surface Area and Porosity, Academic Press, London 1982, Kap. 2+6.

Mit Hilfe des Einsatzes der beschriebenen Katalysatoren entsteht im erfindungsgemäßen Verfahren aus den eingesetzten Isocamphyl-guajakolen bzw. Isocamphylphenolen ein Gemisch isomerer Isocamphyl-cyclohexanole, wie 2-Hydroxy-1-(5-isocamphyl)-cyclohexan, 3-Hydroxy-1-(5-isocamphyl)-cyclohexan, 4-Hydroxy-1-(5-isocamphyl)-cyclohexan. Der Temperaturbereich für das erfindungsgemäße Verfahren beträgt 140 bis 280°C, bevorzugt 180 bis 260°C. Es wird bei einem Wasserstoffdruck von 20 bis 400 bar, bevorzugt 20 bis 350 bar, besonders bevorzugt 100 bis 300 bar, gearbeitet.

Das erfindungsgemäße Verfahren kann mit den im Festbett angeordneten Katalysatoren kontinuierlich in der Rieselphase durchgeführt werden, wobei während des Verfahrensablaufs mindestens die 10fache molare Menge Wasserstoff pro Mol Ausgangsmaterial den Reaktor passiert. Die Hydrierreaktoren können einzelne Hochdruckrohre aus Stahl oder einer Stahllegierung sein, die mit den Formkörpern ganz oder teilweise gefüllt werden, wobei bei größeren Rohrquerschnitten auch die Anwendung der trägerfreien Formkörper auf Horden sowie in Drahtkörben oder ähnlichen Einbauten nützlich sein kann. Weiterhin kann man auch Hochdruck-Rohrbündel innerhalb eines gemeinsamen Mantels anwenden, wobei die Einzelrohre wiederum ganz oder teilweise mit den Katalysatorformkörpern gefüllt werden.

Die verpreßten, trägerlosen Katalysatoren werden durch Wasserstoff reduziert und damit aktiviert. Dies ist grundsätzlich simultan zur Hydrierung des eingesetzten Ausgangsmaterials möglich, wobei jedoch eine längere Einlaufphase erforderlich ist, bevor die Katalysatoren ihre volle Aktivität erreichen und damit die höchstmögliche Raum-Zeit-Ausbeute eintritt. Es ist daher vorteilhaft, den Katalysator vor der Beschickung mit Ausgangsmaterial zu reduzieren. Diese aktivierende Reduktion mit Wasserstoff wird im Temperaturbereich von 180 bis 280°C und im Druckbereich von 20 bis 300 bar durchgeführt. Hierbei wird zunächst durch ein Inertgas, wie Stickstoff, Argon, Methan oder Ethan der zunächst vorhandene Luftsauerstoff vollständig entfernt, bevor dem Inertgas ein Anteil von 10 bis 15 Vol.-% Wasserstoff zugesetzt wird. Das Inertgas ist aus Gründen der guten Verfügbarkeit bevorzugt Stickstoff. Innerhalb eines festgesetzten Zeitraums, beispielsweise von 24 Stunden, wird sodann der Anteil an Inertgas ständig vermindert und schließlich das Inertgas ganz entfernt, so daß mit reinem Wasserstoff aktiviert und reduziert wird. Die Reduktion ist beendet, wenn der Katalysator keinen Wasserstoff mehr verbraucht und infolge dessen kein Reaktionswasser mehr bildet.

Bei der Verfahrensweise in Rieselphase beträgt die Katalysatorbelastung 0,05 bis 1,0 kg, bevorzugt 0,1 bis 0,5 kg Einsatzprodukt pro Liter Katalysator und Stunde. Die eingesetzten Isocamphyl-guajakole bzw. Isocamphyl-phenole können mit einem geeigneten reaktionsinerten Lösungsmittel, beispielsweise aliphatischen Monoalkoholen oder Cyclohexanol in einer Menge von 10 bis 100, bevorzugt 10 bis 40 Gew.-%, bezogen auf das Gewicht des Einsatzproduktes, verdünnt werden.

Die erfindungsgemäß eingesetzten Katalysatoren zeigen sehr hohe Standzeiten; bisher sind 12.000 bis 15.000 Stunden beobachtet worden, bei denen Versuche ohne erkennbares Nachlassen der Aktivität abgebrochen wurden.

Die erhaltenen Reaktionsprodukte sind praktisch frei von aromatischen Anteilen.

Nach der Hydrierung ohne Lösungsmittel können die erhaltenen Isocamphyl-cyclohexanole nach destillativer Entfernung eventuell gebildeter Leichtsieder normalerweise ohne weiteren Reinigungsprozeß, bei der Hydrierung mit Lösungsmittel nach zusätzlicher Abdestillation des Lösungsmittels, weiterverarbeitet werden. Es ist jedoch auch möglich, die erhaltenen Isocamphyl-cyclohexanole destillativ oder mit anderen bekannten physikalischen Trennmethoden zu trennen und aufzukonzentrieren.

### Beispiele

### Beispiel 1

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l durch Tablettierung von Pulvern von Kobalt-, Mangan- und Kupfer-Oxiden hergestellter Formkörper gefüllt. Der Kobaltgehalt der Tabletten lag bei 54 Gew.-%, der Mangangehalt bei 15 Gew.-% und der Kupfergehalt bei 0,2 Gew.-%. Die Tabletten hatten bei einer Zylinderhöhe von 5 mm und einem Durchmesser von 5 mm eine Druckfestigkeit von 415 N/cm² auf die plane Zylinderoberfläche und von 120 N auf die gewölbte Formkörperoberfläche sowie eine innere Oberfläche von 165 m²/g.

Die Tabletten wurden zunächst 6 Stunden im Stickstoffstrom getrocknet (Temperatur: max. 200°C, Menge: 5 Nm³ N₂/h). Die Aktivierung erfolgte unter einem Stickstoffdruck von 200 bar bei einer Temperatur zwischen 180 und 280°C, wobei dem Stickstoff allmählich Wasserstoff zugemischt wurde, dessen Zumischungsanteil anfangs 10 bis 15 Vol.-% betrug. Innerhalb von 24 Stunden wurde der Stickstoffanteil der Gasmischung mehr und mehr vermindert, bis schließlich reiner Wasserstoff den Reaktor durchströmte. Die Aktivierung war beendet, sobald sich kein Reaktionswasser im nachgeschalteten Abscheider mehr ansammelte.

Nach der Aktivierung des Katalysators wurde der Wasserstoffdruck im Reaktorsystem auf 300 bar erhöht. Anschließend wurden stündlich 170 g einer 38 gew.-%igen Lösung eines Gemisches aus Isocamphyl-guajakolen, wie es bei der Alkylierung von Camphen mit Guajakol anfällt, in Cyclohexanol gemeinsam mit 1,5 Nm³ Wasserstoff unter einem Druck von 300 bar von oben nach unten durch das Hochdruckrohr gepumpt, wobei die zu hydrierende Mischung vor Eintritt in das Hochdruckrohr in einem vorgeschalteten elektrisch beheizten Wärmeaustauscher auf eine Temperatur von 190°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Reaktionssystem zurückgeführt wurde, getrennt.

Nach weiterer Abkühlung auf eine Temperatur <30°C und Entspannung auf Normaldruck wurde das Reaktionsprodukt UV-spektroskopisch untersucht.

Es wurde festgestellt, daßa der Restaromatanteil bei weniger als 0,1 Gew.-% lag.

Nach dem Abdestillieren des Lösungsmittels wurde ein glasklares viskoses Öl erhalten, das die isomeren Isocamphylverbindungen
2-Hydroxy-1-(5-isocamphyl)-cyclohexan,
3-Hydroxy-1-(5-isocamphyl)-cyclohexan und
4-Hydroxy-1-(5-isocamphyl)-cyclohexan
im Mischungsverhältnis von ca. 3:1:2 enthielt und angenehm nach Sandelholz duftete.

Der Katalysator war nach einer Laufzeit von 10 106 Stunden unverändert wirksam.

### Beispiel 2

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l durch Tablettierung von Pulvern von Kobalt-, Mangan-, Barium- und Kupfer-Oxiden hergestellter Formkörper gefüllt. Der Kobaltgehalt der Tabletten lag bei 53 Gew.-%, der Mangangehalt bei 14 Gew.-%, der Bariumgehalt bei 0,9 Gew.-% und der Kupfergehalt bei 0,2 Gew.-%. Die Tabletten hatten bei einer Zylinderhöhe von 5 mm und einem Durchmesser von 5 mm eine Druckfestigkeit von 420 N/cm² auf die plane Zylinderoberfläche und von 128 N auf die gewölbte Formkörperfläche sowie eine innere Oberfläche von 168 m²/g.

Die Tabletten wurden zunächst 6 Stunden im Stickstoffstrom getrocknet (Temperatur: max. 200°C, Menge: 5 Nm³ N₂/h). Die Aktivierung erfolgte unter einem Stoffdruck von 200 bar bei einer Temperatur zwischen 180 und 280°C, wobei dem Stickstoff allmählich Wasserstoff zugemischt wurde, dessen Zumischungsanteil anfangs 10 bis 15 Vol.-% betrug. Innerhalb von 24 Stunden wurde der Stickstoffanteil der Gasmischung mehr und mehr vermindert, bis schließlich reiner Wasserstoff den Reaktor durchströmte. Die Aktivierung war beendet, sobald sich kein Reaktionswasser im nachgeschalteten Abscheider mehr ansammelte.

Nach der Aktivierung des Katalysators wurde der Wasserstoffdruck im Reaktorsystem auf 300 bar erhöht. Anschließend wurden stündlich 180 g einer 38 gew.-%igen Lösung eines Gemisches aus Isocamphyl-guajakolen, wie es bei der Alkylierung von Camphen mit Guajakol anfällt, in Cyclohexanol gemeinsam mit 1,5 Nm³ Wasserstoff unter einem Druck von 300 bar von oben nach unten durch das Hochdruckrohr gepumpt, wobei die zu hydrierende Mischung vor Eintritt in das Hochdruckrohr in einem vorgeschalteten elektrisch beheizten Wärmeaustauscher auf eine Temperatur von 210°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur >60°C abgekühlt und in einem Gasabscheider vom überschüssigen Wasserstoff, der wieder in das Reaktionssystem zurückgeführt wurde, getrennt.

Nach weiterer Abkühlung auf eine Temperatur <30°C und Entspannung auf Normaldruck wurde das Reaktionsprodukt UV-spektroskopisch untersucht.

Es wurde festgestellt, daß der Restaromatanteil bei weniger als 0,1 Gew.-% lag.

Nach dem Abdestillieren des Lösungsmittels wurde ein glasklares viskoses Öl erhalten, das die isomeren Isocamphylverbindungen
2-Hydroxy-1-(5-isocamphyl)-cyclohexan,
3-Hydroxy-1-(5-isocamphyl)-cyclohexan und
4-Hydroxy-1-(5-isocamphyl)-cyclohexan
im Mischungsverhältnis von ca. 3:1:3 enthielt und angenehm nach Sandelholz duftete.

Der Katalysator war nach einer Laufzeit von 4 026 Stunden unverändert wirksam.

## Patentansprüche

1. Kontinuierliches Verfahren zur Herstellung von Isocamphyl-cyclohexanolen aus Verbindungen, die das Kohlenstoffgerüst der Isocamphyl-guajakole bzw. Isocamphyl-phenole besitzen, durch katalytische Hydrierung mit Wasserstoff bei erhöhter Temperatur und erhöhtem Druck, dadurch gekennzeichnet, daß als Katalysatoren trägerfreie Formkörper verwendet werden, die durch Reduktion von Formkörpern aus verpreßten Pulvern von Kobalt-, Mangan- und Kupfer(hydr)oxiden und gegebenenfalls einem oder mehreren (Hydr)Oxiden der Erdalkalimetalle erhältlich sind.

2. Verfahren nach Anspruch 1, dadurch gekennzeichnet, daß die für die Reduktion zu verwendenden Formkörper aus verpreßtem Metall(hydr)oxidpulvern (jeweils berechnet als Metall) 40 bis 65 Gew.-% Kobalt, 10 bis 20 Gew.-% Mangan, 0,1 bis 0,5 Gew.-% Kupfer und 0 bis 5 Gew.-% Erdalkalimetall enthalten, wobei sich die Prozentangaben auf die Gesamtmenge Metalloxid-Pulvergemisch beziehen.

3. Verfahren nach Anspruch 1, dadurch gekennzeichnet, daß ein oder mehrere (Hydr)Oxide der Erdalkalimetalle in dem verpreßten (Hydr)Oxidpulver vorliegen können, wobei für den Fall des Vorliegens mehrerer (Hydr)Oxidpulver jedes dieser (Hydr)Oxidpulver in einer Menge vorliegt, die nicht kleiner als 20 Gew.-% und nicht größer als 80 Gew.-% des Gesamtgehalts von bis zu 5 Gew.-% ist.

4. Verfahren nach Anspruch 1, dadurch gekennzeichnet, daß die Formkörper aus verpreßten Metall(hydr)oxidpulvern eine Druckfestigkeit von 300 bis 800 N/cm ² auf die Formkörperoberfläche (gemessen nach DIN 50 106) besitzen.

5. Verfahren nach Anspruch 1, wonach die Formkörper aus verpreßten Metall(hydr)oxidpulvern eine innere Oberfläche von 30 bis 200m²/g aufweisen.

6. Verfahren nach Anspruch 1, wobei der Wasserstoffdruck 20 bis 400 bar beträgt.

7. Verfahren nach Anspruch 1, wobei die Hydriertemperatur 140 bis 240°C beträgt.

8. Verfahren nach Anspruch 1, dadurch gekennzeichnet, daß während des Verfahrens pro Mol Ausgangsmaterial mindestens die 10fache molare Menge Wasserstoff den Reaktor passiert.

9. Verfahren nach Anspruch 1, dadurch gekennzeichnet, daß kontinuierlich in der Rieselphase an fest angeordneten Katalysatoren gearbeitet wird und eine Katalysatorbelastung von 0,05 bis 1,0 kg Ausgangsprodukt, pro Liter Katalysator und Stunde eingestellt wird.

10. Verfahren nach Anspruch 1, dadurch gekennzeichnet, daß die Katalysatoren vor ihrem Einsatz durch Behandlung mit Waserstoff bei 180 bis 280°C und 20 bis 300 bar Wasserstoffdruck reduziert werden, wobei der Wasserstoff zu Beginn der Reduktion als Wasserstoff/Inertgas-Gemisch eingesetzt wird und der Inertgasanteil im Verlauf der Reduktion vollständig entfernt wird.

11. Verfahren nach Anspruch 1, dadurch gekennzeichnet, daß das Einsatzprodukt mit 10 bis 100 Gew.-% eines reaktionsinerten Lösungsmittels, bezogen auf das Einsatzprodukt, verdünnt wird.

## Claims

1. Continuous process for preparing isocamphyl-cyclohexanols from compounds which have the carbon skeleton of the isocamphyl-guaiacols or isocamphylphenols by catalytic hydrogenation with hydrogen at elevated temperature and elevated pressure, characterized in that support-free moulded bodies are used as catalysts and are obtainable by reduction of moulded bodies made of compressed powders of cobalt, manganese and copper (hydr)oxides and optionally one or more (hydr)oxides of the alkaline earth metals.

2. Process according to Claim 1, characterized in that the moulded bodies to be used for the reduction and composed of compressed metal (hydr)oxide powders contain (calculated as metal in each case) 40 to 65% by weight of cobalt, 10 to 20% by weight of manganese, 0.1 to 0.5% by weight of copper and 0 to 5% by weight of alkaline earth metal, the percentages relating to the total amount of metal oxide powder mixture.

3. Process according to Claim 1, characterized in that one or more (hydr)oxides of the alkaline earth metals may be present in the compressed (hydr)oxide powder, in which case, if a plurality of (hydr)oxide powders is present, each of said (hydr)oxide powders is present in an amount which is not less than 20% by weight and not greater than 80% by weight of the total content of up to 5% by weight.

4. Process according to Claim 1, characterized in that the moulded bodies made of compressed metal (hydr)oxide powders have a compressive strength of 300 to 800 N/cm² on the moulded body surface (measured according to DIN 50 106).

5. Process according to Claim 1, according to which the moulded bodies made of compressed metal (hydr)oxide powders have an internal surface of 30 to 200 m²/g.

6. Process according to Claim 1, the hydrogen pressure being 20 to 400 bar.

7. Process according to Claim 1, the hydrogenation temperature being 140 to 240°C.

8. Process according to Claim 1, characterized in that at least ten times the molar amount of hydrogen passes through the reactor per mole of starting material during the process.

9. Process according to Claim 1, characterized in that fixed catalysts are continuously employed in the free-flowing phase and a weight hourly space velocity of 0.05 to 1.0 kg of starting product per litre of catalyst and per hour is established.

10. Process according to Claim 1, characterized in that the catalysts are reduced by treatment with hydrogen at 180 to 280°C and 20 to 300 bar hydrogen pressure before they are used, the hydrogen being used as hydrogen/inert-gas mixture at the beginning of the reduction and the inert-gas component being removed completely in the course of the reduction.

11. Process according to claim 1, characterized in that the feedstock is diluted with 10 to 100% by weight of a solvent which is inert to the reaction, relative to the feedstock.

## Revendications

1. Procédé continu pour la préparation d'isocamphyl-cyclohexanols à partir de composés possédant le squelette carboné des isocamphyl-gaïacols ou des isocamphyl-phénols, par hydrogénation catalytique à température et pression élevées, caractérisé en ce que l'on utilise en tant que catalyseurs des corps moulés sans supports obtenus par réduction de corps moulés par compression de poudres d'(hydr)oxydes de cobalt, de manganèse et de cuivre et le cas échéant d'un ou plusieurs (hydr)oxydes de métaux alcalino-terreux.

2. Procédé selon la revendication 1, caractérisé en ce que les corps soumis à la réduction, moulés par compression de poudres d'(hydr)oxydes métalliques contiennent (dans chaque cas exprimé en métal) 40 à 65 % en poids de cobalt, 10 à 20 % en poids de manganèse, 0,1 à 0,5 % en poids de cuivre et 0 à 5 % en poids de métaux alcalino-terreux, ces pourcentages se rapportant à la quantité totale du mélange des poudres d'oxydes métalliques.

3. Procédé selon la revendication 1, caractérisé en ce que les poudres d'(hydr)oxydes comprimées peuvent contenir un ou plusieurs (hydr)oxydes de métaux alcalino-terreux et dans le cas de la présence de plusieurs poudres d'(hydr)oxydes, chacune de ces poudres d'(hydr)oxydes est présente en quantité non inférieure à 20 % en poids et non supérieure à 80 % en poids par rapport à la teneur totale allant jusqu'à 5 % en poids.

4. Procédé selon la revendication 1, caractérisé en ce que les corps moulés par compression de poudres d'(hydr)oxydes métalliques ont une résistance à la compression de 300 à 800 N/cm² sur la surface des corps moulés (mesure selon norme allemande DIN 56 106).

5. Procédé selon la revendication 1, dans lequel les corps moulés par compression de poudres d'(hydr)oxydes métalliques ont une surface interne de 30 à 200 m²/g.

6. Procédé selon la revendication 1, dans lequel la pression d'hydrogène va de 20 à 400 bars.

7. Procédé selon la revendication 1, dans lequel la température d'hydrogénation est de 140 à 240°C.

8. Procédé selon la revendication 1, caractérisé en ce que, au cours des opérations, il passe dans le réacteur au moins 10 fois la quantité molaire d'hydrogène par mole de composant de départ.

9. Procédé selon la revendication 1, caractérisé en ce que l'on opère en continu et en phase ruisselante sur des catalyseurs en disposition fixe et on règle à une charge de catalyseur de 0,05 à 1,0 kg de produit de départ par litre de catalyseur et par heure.

10. Procédé selon la revendication 1, caractérisé en ce que, avant mise en oeuvre, les catalyseurs sont réduits par l'hydrogène à des températures de 180 à 280°C et des pressions d'hydrogène de 20 à 300 bars, l'hydrogène étant mis en oeuvre en début de réduction sous forme d'un mélange hydrogène/gaz inerte d'où l'on élimine totalement le gaz inerte en cours de réduction.

11. Procédé selon la revendication 1, caractérisé en ce que le produit mis en oeuvre est dilué par 10 % à 100 % de son poids d'un solvant inerte dans la réaction.
